# EUROPEAN PATENT APPLICATION

(11) **EP 4 687 146 A1**
(43) Date of publication of application: **04.02.2026**
(21) Application number: 25191426.3
(22) Date of filing: 23.07.2025
(51) Int. Cl.: G16C 20/10, G16C 20/70, G16C 20/50

(54) **PREDICTING MOLECULE SYNTHESIZABILITY USING MACHINE LEARNING**

(30) Priority: 29.07.2024 GR 20240100529; 15.11.2024 GR 20240100815
(71) Applicant: Isomorphic Labs Limited, London EC2M 4RB (GB)
(72) Inventor: TIGAS, Panagiotis, London, EC2M 4RB (GB); SMITH, Raymond Wensley, London, EC2M 4RB (GB)
(74) Representative: Marks & Clerk GST

(57) **Abstract**

Methods, systems, and apparatus, including computer programs encoded on a computer storage medium, for predicting synthesis complexity. In one aspect, a method comprises: receiving data characterizing a molecule; processing a model input that comprises the data characterizing the molecule, using a retrosynthesis featurization machine learning model and in accordance with values of a set of retrosynthesis featurization machine learning model parameters, to generate a feature array comprising a plurality of features of a retrosynthesis tree for the molecule; and processing a model input that comprises the feature array comprising the plurality of features of the retrosynthesis tree for the molecule, using a synthesizability prediction machine learning model and in accordance with values of a set of synthesizability prediction machine learning model parameters, to generate a synthesizability score that characterizes a synthesis complexity of the molecule.

## Description

### BACKGROUND

This specification relates to processing data using machine learning models.

Machine learning models receive an input and generate an output, e.g., a predicted output, based on the received input. Some machine learning models are parametric models and generate the output based on the received input and on values of the parameters of the model.

Some machine learning models are deep models that employ multiple layers of models to generate an output for a received input. For example, a deep neural network is a deep machine learning model that includes an output layer and one or more hidden layers that each apply a nonlinear transformation to a received input to generate an output.

Chemical synthesizability is the feasibility with which a given target molecule can be synthesized. For instance, a molecule is considered to be more synthesizable if it can be produced using readily available and inexpensive intermediate molecules. Retrosynthesis is a reverse-engineering analysis of a target molecule that can be used to deconstruct a target molecule into simpler precursor molecules by identifying key chemical bonds suitable for strategic disconnection, thereby reducing molecular complexity for efficient synthesis.

### SUMMARY

This specification describes a system implemented as computer programs on one or more computers in one or more locations that can generate a synthesizability score for a molecule. In particular, the system can process data representing the molecular structure of a molecule using a retrosynthesis featurization machine learning model to generate a retrosynthesis feature array that characterizes the retrosynthesis tree of the molecule. The system can further process the retrosynthesis feature array using a synthesizability prediction machine learning model to generate the synthesizability score.

Throughout this specification, a "molecule" can refer to a collection of atoms which are bonded together through chemical bonds. A molecule can be represented in any of a variety of possible ways, e.g., as a sequence of characters, e.g., a Simplified Molecular Input Line Entry System (SMILES) string.

A "tree" (e.g., a retrosynthesis tree) can refer to a hierarchical data structure that includes a set of nodes and a set of branches. The set of nodes includes a single, designated "root" node, from which all other nodes descend. Each branch connects a "parent" node to one or more respective "child" nodes in the tree. Each node in the tree, except for the root node, is connected by exactly one branch to a unique parent node, thereby forming a parent-child relationship. A "leaf" node in a tree refers to a node without any child nodes. A tree is acyclic, i.e., does not include any cycles or closed loops within its structure.

A "retrosynthesis tree" for a target molecule can refer to a tree that defines a synthesis pathway for the target molecule. More specifically, each node in the retrosynthesis tree can represent a respective molecule, with the root node representing the target molecule. Each non-leaf node has one or more child nodes that each represent a respective reactant molecule involved in a chemical reaction that produces the molecule represented by the non-leaf node. Leaf nodes can represent starting materials (reactant molecules) for the synthesis pathway.

A "complete" retrosynthesis tree can refer to a retrosynthesis tree where each leaf node of the retrosynthesis tree represents a respective molecule that is included in a predefined library of precursor molecules. An "incomplete" retrosynthesis tree can refer to a retrosynthesis tree that is not complete, i.e., that includes at least one leaf node representing a molecule that is not included in the predefined library of precursor molecules.

A "library of precursor molecules" can refer to any predefined set of molecules. In some cases, the library of precursor molecules can be a set of molecules that are defined to be available for use by the system for synthesizing the target molecule. These can comprise, e.g., molecules that are available commercially or that are straightforward to make, or that each have desirable manufacturability properties, e.g., that are each known to be manufacturable with greater than a defined yield, or within a defined time period, or within a defined budget, and so forth.

An "embedding" refers to an ordered collection of numerical values, e.g., a vector, matrix, or other tensor of numerical values.

According to one aspect, there is provided a computer-implemented method comprising: receiving data characterizing a molecule. The method involves processing a model input that comprises the data characterizing the molecule, e.g. characterizing a chemical structure of the molecule, using a (trained) retrosynthesis featurization machine learning model and in accordance with values of a set of retrosynthesis featurization machine learning model parameters, to generate a feature array comprising a plurality of features of a retrosynthesis tree for the molecule. Some examples of features are described later. The method further involves processing a model input that comprises the feature array comprising the plurality of features of the retrosynthesis tree for the molecule, using a (trained) synthesizability prediction machine learning model and in accordance with values of a set of synthesizability prediction machine learning model parameters, to generate a synthesizability score that characterizes a synthesis complexity, e.g., a synthesis feasibility, of the molecule. Techniques for training the machine learning models are described later.

In some implementations, the data characterizing the molecule comprises data defining a chemical structure of the molecule.

In some implementations, the data defining the chemical structure of the molecule comprises a Simplified Molecular Input Line Entry System (SMILES) string.

In some implementations, the retrosynthesis featurization machine learning model has been trained by operations comprising: obtaining a set of training examples. In implementations each training example corresponds to a respective training molecule and comprises: (i) a training model input characterizing the training molecule, and (ii) a target feature array comprising a plurality of features of a retrosynthesis tree for the training molecule; and training the retrosynthesis featurization machine learning model on the set of training examples.

Also or instead, for a plurality of training examples in the set of training examples, the target feature array of the training example is generated by operations comprising: processing data characterizing the training molecule using a retrosynthesis planner to generate a predicted retrosynthesis tree for the training molecule; and processing the predicted retrosynthesis tree for the training molecule to generate the target feature array of the training example.

In some implementations, a plurality of training examples in the set of training examples has been generated by performing operations comprising obtaining a collection of baseline, e.g. known, retrosynthesis trees, and generating a plurality of new retrosynthesis trees by programmatically assembling baseline retrosynthesis trees from the collection of baseline retrosynthesis trees. In general, this programmatic assembling involves combining the baseline retrosynthesis trees according to a defined set of rules or structured method. In some implementations this involves generating a respective training example for each new retrosynthesis tree, comprising, for each new retrosynthesis tree: generating a training model input for the training example based on a training molecule represented by a root node of the retrosynthesis tree; and generating a target feature array for the training example by processing the new retrosynthesis tree to determine the plurality of features for the training.

In some implementations, obtaining the collection of baseline retrosynthesis trees comprises identifying the collection of baseline retrosynthesis trees by scraping a corpus of data using natural language processing techniques.

In some implementations, programmatically assembling baseline retrosynthesis trees from the collection of baseline retrosynthesis trees comprises, for each new retrosynthesis tree, searching a space of possible combinations of baseline retrosynthesis trees having leaf nodes that each represent a molecule included in a predefined library of precursor molecules to identify the new retrosynthesis tree, wherein the search is performed to optimize an objective function that measures a penalty associated with the new retrosynthesis tree.

In some implementations, searching the space of possible combinations of baseline retrosynthesis trees further comprises: identifying a first baseline retrosynthesis tree comprising a root node and one or more leaf nodes; identifying a plurality of second baseline retrosynthesis trees having root nodes that each represent a same molecule as a corresponding leaf node of the first baseline retrosynthesis tree; determining, for each of the plurality of second baseline retrosynthesis tree, a penalty that would result from attaching the second baseline retrosynthesis tree to the first baseline retrosynthesis tree at the corresponding leaf node of the first baseline retrosynthesis tree; and selecting a second baseline retrosynthesis tree for attachment to the first baseline retrosynthesis tree at each of the corresponding leaf nodes based on the penalties.

In some implementations, the method iteratively identifies the first baseline retrosynthesis tree and the plurality of the second baseline retrosynthesis trees for each of a set of root nodes and corresponding leaf nodes of the new retrosynthesis tree. This can be referred to as dynamic programming.

In some implementations, the penalty characterizes one or more of: a depth of the new retrosynthesis tree, a measure of ease of procurement of the molecules represented by the nodes in the new retrosynthesis tree, or a yield of the reactions in a synthesis pathway represented by the new retrosynthesis tree.

In some implementations, processing data characterizing the training molecule involves using a retrosynthesis planner to generate a predicted retrosynthesis tree for the training molecule comprises: processing the data characterizing the training molecule using the retrosynthesis planner to generate a plurality of predicted retrosynthesis trees for the training molecule; and wherein processing the predicted retrosynthesis tree for the training molecule to generate the target feature array of the training example comprises: processing the plurality of predicted retrosynthesis trees to generate the target feature array of the training example.

In some implementations, processing the plurality of predicted retrosynthesis trees to generate the target feature array of the training example comprises: processing each of the plurality of predicted retrosynthesis trees to generate a corresponding feature array; and aggregating the feature arrays for the plurality of predicted retrosynthesis trees to generate the target feature array.

In some implementations, processing the plurality of predicted retrosynthesis trees to generate the target feature array of the training example comprises: selecting a set of at least one but fewer than all of the plurality of predicted retrosynthesis trees; and generating the target feature array based on only the selected predicted retrosynthesis trees.

In some implementations, selecting the set of at least one but fewer than all of the plurality of predicted retrosynthesis trees comprises: ranking the plurality of retrosynthesis trees based on one or more criteria to select the set; or clustering the plurality of retrosynthesis trees to select the set based at least on a measure of diversity of synthesis.

In some implementations, the retrosynthesis planner is implemented as a machine learning model having a set of machine learning model parameters that are trained by a machine learning training technique.

In some implementations, training the retrosynthesis featurization machine learning model on the set of training examples comprises, for each training example: training the retrosynthesis featurization machine learning model to reduce a discrepancy between: (i) the target feature array of the training example, and (ii) a predicted feature array generated by processing the training model input of the training example using the retrosynthesis featurization machine learning model.

In some implementations, the retrosynthesis featurization machine learning model comprises a neural network.

In some implementations, the retrosynthesis featurization machine learning model comprises a graph neural network.

In some implementations, processing the model input that comprises the data characterizing the molecule, using the retrosynthesis featurization machine learning model and in accordance with values of the set of retrosynthesis featurization machine learning model parameters, to generate the feature array comprising the plurality of features of the retrosynthesis tree for the molecule comprises: generating data defining a graph representing the molecule, wherein the graph comprises a set of nodes and a set of edges, wherein each edge in the set of edges connects a respective pair of nodes from the set of nodes, wherein each node in the set of nodes is associated with a respective set of node features; processing, for each node in the graph, the set of node features of the node using an encoder block of the graph neural network to generate an embedding of the node; processing the embeddings of the nodes in the graph by a plurality of message passing neural network layers of the graph neural network to generate a respective final embedding for each node in the graph; and processing the final embeddings of the nodes in the graph using a decoder block of the graph neural network to generate the feature array.

In some implementations, the synthesizability prediction machine learning model has been trained by operations comprising: obtaining a set of training examples, wherein each training example corresponds to a respective training molecule and comprises: (i) a training model input comprising a feature array including a plurality of features of a retrosynthesis tree for the training molecule, and (ii) a target synthesizability score for the training molecule; and training the retrosynthesis featurization machine learning model on the set of training examples.

In some implementations, training the synthesizability prediction machine learning model on the set of training examples comprises, for each training example: training the synthesizability prediction machine learning model to reduce a discrepancy between: (i) the target synthesizability score of the training example, and (ii) a predicted synthesizability score generated by processing the training model input of the training example using the synthesizability prediction machine learning model.

In some implementations, the synthesizability prediction machine learning model comprises a support vector machine.

In some implementations, the synthesizability prediction machine learning model comprises a differentiable machine learning model.

In some implementations, the retrosynthesis featurization machine learning model and the synthesizability prediction machine learning model have been jointly trained by operations comprising: obtaining a set of training examples, wherein each training example corresponds to a respective training molecule and comprises: (i) a training model input comprising data characterizing the training molecule, and (ii) a target synthesizability score; and training the retrosynthesis featurization machine learning model and the synthesizability prediction machine learning model to reduce a discrepancy between: (i) the target synthesizability score of the training example, and (ii) a predicted synthesizability score.

In some implementations, training the retrosynthesis featurization machine learning model and the synthesizability prediction machine learning model comprises: processing each of the training model inputs using the retrosynthesis featurization machine learning model to generate respective predicted feature arrays; processing each of the respective predicted feature arrays using the synthesizability prediction machine learning model to generate respective predicted synthesizability scores; and updating values of a set of retrosynthesis featurization machine learning model parameters and values of a set of synthesizability prediction machine learning model parameters in accordance with reducing the discrepancy between: (i) the respective target synthesizability score, and (ii) the respective predicted synthesizability scores.

In some implementations, the method further comprises using the synthesizability score to perform classifier guidance for a generative model that is configured to generate output molecules.

In some implementations, the generative model is configured to generate a ligand molecule for binding to a target protein molecule.

In some implementations, the generative model is implemented as a generative diffusion model.

In some implementations, using the synthesizability score to perform classifier guidance for the generative model encourages the generative model to generate output molecules that have at least a threshold level of synthesizability.

In some implementations, using the synthesizability score to perform classifier guidance for the generative model comprises modifying an intermediate layer representation of the generative model.

In some implementations, the feature array further comprises: a set of one or more features characterizing the retrosynthesis tree; and a set of one or more biophysical and biochemical properties of the molecule.

In some implementations, the set of one or more features characterizing the retrosynthesis tree comprises one or more of: a depth indicative of a longest path from a root node of the retrosynthesis tree to any leaf node of the retrosynthesis tree, a number of nodes of the retrosynthesis tree, a probability of a reaction represented by the retrosynthesis tree, a reactivity indicator with reference to the retrosynthesis tree, a stability indicator with reference to the retrosynthesis tree, and a completeness of the retrosynthesis tree indicative of each molecule represented by a leaf node of the retrosynthesis tree being included in a predefined library of precursor molecules. As an example, the completeness can be indicated by a binary value, or a binary value per molecule, or a scalar value for the retrosynthesis tree indicating a degree of the completeness.

In some implementations, the set of one or more biophysical and biochemical properties comprises one or more of molecular weight, topological polar surface area, formal charge (e.g., of one or more atoms of the molecule), number of hydron acceptors and donors, number of rotatable bonds, number of aromatic and aliphatic rings, number of carbon-carbon single bonds, number of heavy atoms, or number of heteroatoms properties.

According to another aspect, there is provided a computer-implemented method comprising: receiving a collection of candidate molecules; generating a respective synthesizability score for each of the candidate molecules using the methods described herein; and selecting one or more of the candidate molecules for physical synthesis based at least in part on the respective synthesizability scores. The selected molecule(s) can be synthesized manually and/or automatically, e.g. by a robot.

In some implementations, the method further comprises physically synthesizing the one or more candidate molecules selected for synthesis.

In some implementations, the method further comprises performing physical experiments to determine one or more properties of the physically synthesized one or more candidate molecules.

According to another aspect, there is provided a system comprising one or more computers and one or more storage devices storing instructions that are operable, when executed by the one or more computers, to cause the one or more computers to perform operations of the methods described herein.

According to another aspect, there are provided one or more non-transitory computer storage media encoded with a computer program, the program comprising instructions that are operable, when executed by data processing apparatus, to cause the data processing apparatus to perform the methods described herein.

Particular embodiments of the subject matter described in this specification can be implemented so as to realize one or more of the following advantages.

The system can process a molecule to generate a synthesizability score that characterizes a feasibility of synthesizing the molecule, e.g., based on one or more of: the complexity and number of steps required to synthesize the molecule (including the types of chemical reactions involved and their respective yields), the scalability of the synthesis process (e.g., how readily the synthesis process can be scaled from laboratory scale production to industrial scale production), the stability of intermediate compounds throughout the synthesis process, the reaction yield of the synthesis process, the reaction conditions required to carry out the synthesis process, and so forth.

A retrosynthesis tree for a molecule encodes information and features that are highly relevant to predicting the synthesizability of the molecule. However, computationally generating a retrosynthesis tree for a molecule can be a computationally expensive and slow process, often requiring an analysis of potential pathways at each node representing a reactant. Therefore an approach for predicting synthesizability that relies on generating a retrosynthesis tree for a molecule in order to predict the synthesizability of the molecule may be computationally infeasible, particularly in applications that require generating large numbers of synthesizability scores, e.g., performing classifier guidance for a generative model (as will be described in more detail below).

Another approach to predicting synthesizability for a molecule can involve processing data characterizing the molecule using a neural network that is trained from end-to-end to directly generate a synthesizability score for the molecule. However, predicting synthesizability directly from data characterizing the chemical structure of a molecule is a complex prediction task that may require a complex neural network architecture with a large number of parameters, e.g., a million or more parameters. However, neural network with complex architectures and large numbers of parameters generally must be trained on large sets of labeled training data, i.e., that associate individual molecules with corresponding synthesizability scores. However, training data that labels molecules with synthesizability scores is scarce and often not present in quantities sufficient to enable training of neural networks with complex architectures and large numbers of parameters.

The system described in this specification addresses these issues to enable rapid and accurate generation of synthesizability scores for molecules. In particular, the system can generate a synthesizability score for a molecule by a two-stage process. In the first stage, a first machine learning model (referred to herein as a "retrosynthesis featurization machine learning model") processes data characterizing a molecule to generate a feature array with features characterizing a retrosynthesis tree for the molecule. In the second stage, a second machine learning model (referred to herein as a "synthesizability prediction machine learning model") processes the array of retrosynthesis tree features to generate a synthesizability score for the molecule. The system can thus leverage relevant features of a retrosynthesis tree for a molecule, i.e., as generated by the retrosynthesis featurization machine learning model, without actually generating the entire retrosynthesis tree.

The system can train the retrosynthesis featurization machine learning model on a set of training data that is generated (at least in part) by computationally generating retrosynthesis trees for molecules and extracting feature arrays from the computationally generated retrosynthesis trees. Generally, training a machine learning model such as the retrosynthesis featurization machine learning model to effectively perform a machine learning task can require a large quantity of high quality training data.

Computational methods for generating predicted retrosynthesis trees can generate large quantities of training data, but in some cases, these computationally generated trees can include "hallucinations" that include various inaccuracies, e.g., by defining non-physical synthesis pathways that include reactions with non-reactive reactants. Training the retrosynthesis featurization machine learning model on hallucinations can compromise the training of the retrosynthesis featurization machine learning model, e.g., by preventing the training from converging and by reducing the accuracy of the trained retrosynthesis featurization machine learning model.

The system of this specification can programmatically assemble retrosynthesis trees as training examples from a collection of baseline retrosynthesis trees, e.g., one-step reaction retrosynthesis trees, which addresses these issues since there is a high confidence that the new retrosynthesis trees are accurate, i.e., and not hallucinated trees, since they were generated by aggregating experimentally verified baseline trees.

Furthermore, the combinatorics of aggregating baseline retrosynthesis trees provides for an iterative approach to generating very large numbers of new retrosynthesis trees by programmatic aggregation, even if the number of baseline retrosynthesis trees is not large. More specifically, the system can use a single baseline retrosynthesis tree to generate multiple new retrosynthesis trees by identifying different second baseline retrosynthesis trees, and continuing to add additional baseline retrosynthesis trees to the new retrosynthesis tree.

Moreover, the system can leverage the parent-child node structure to efficiently build a new retrosynthesis tree using dynamic programming, e.g., by breaking down the generation of the whole new retrosynthesis tree into subcomponents involving identifying a next baseline retrosynthesis tree for each leaf node of the previous retrosynthesis tree. In particular, the system can identify a first baseline retrosynthesis tree including a root node and one or more leaf nodes, select one or more second baseline retrosynthesis trees with root nodes representing the same molecule as a corresponding leaf node of the first baseline retrosynthesis tree, and recursively select additional baseline retrosynthesis trees with root nodes representing the same molecule as a corresponding leaf node of the previous retrosynthesis tree.

Therefore, generating training data for training retrosynthesis featurization model by programmatically aggregating baseline trees solves a technical problem arising in training the retrosynthesis featurization machine learning model since the system provides for the efficient generation of a large number of new retrosynthesis trees. Moreover, the system can decrease the use of computational resources necessary to generate the training data since there is no need to implement an additional filtering step to identify spuriously generated retrosynthesis trees representing impossible chemical reactions. Instead, the system can programmatically assemble the new retrosynthesis trees as an operational set of training data without the requirement for further postprocessing.

In the described approach, retrosynthesis trees need only be generated once, and only during training. Following training, the retrosynthesis featurization machine learning model can directly generate arrays of retrosynthesis tree features without generating full retrosynthesis trees. Generating the array of retrosynthesis tree features requires less computational resources, e.g., memory and computing power, and is faster relative to generating a full retrosynthesis tree for a molecule, thereby enabling an increase in efficiency of generating the synthesizability score for each molecule. In particular, in applications such as using synthesizability scores to perform classifier guidance, which can require generating a large number of synthesizability scores, e.g., at each of a large number of denoising iterations when generating molecules using a generative diffusion model, this can enable a significant reduction of computational resources with a corresponding gain in computational efficiency.

Additionally, as described above, the system can use a two-stage model to distill the complexity of predicting synthesizability into a distinct two-stage process. By simplifying the complex prediction into a two-stage prediction, in which the second prediction relies on the output of the first prediction, the system can use two models with smaller architectures and therefore a sum total fewer parameters than would have been necessary to predict the synthesizability score in a unified architecture. Training two smaller models with fewer parameters requires less computational resources (e.g., memory and computing power) than training a larger, more complex model for the same task.

Furthermore, the system can predict the synthesizability score from a feature array that characterizes the retrosynthesis tree of the molecule, e.g., without the need to generate a number of retrosynthesis trees for the molecule. Predicting a feature array that characterizes the retrosynthesis tree reduces computational resources relative to generating one or more retrosynthesis trees for a target molecule, which can be a computationally-expensive process, e.g., a semi-exhaustive search, that can take a significant amount of time to perform.

The details of one or more embodiments of the subject matter of this specification are set forth in the accompanying drawings and the description below. Other features, aspects, and advantages of the subject matter will become apparent from the description, the drawings, and the claims.

### BRIEF DESCRIPTION OF THE DRAWINGS

FIG. 1 illustrates an overview of generating a synthesizability score for a molecule.
FIG. 2 is a block diagram of an example synthesizability prediction system that includes a retrosynthesis featurization machine learning model and a synthesizability prediction machine learning model.
FIG. 3 depicts an example of processing retrosynthesis trees to generate corresponding feature arrays.
FIG. 4 is a flow diagram of an example process for generating a synthesizability score.
FIG. 5 is a flow diagram of an example process for training the retrosynthesis featurization machine learning model.
FIG. 6 is a flow diagram of an example process for training the synthesizability machine learning model.
FIG. 7 is a flow diagram of an example process for jointly training the retrosynthesis featurization and synthesizability machine learning models.
FIG. 8 is a flow diagram of an example process for generating a training example by programmatically assembling baseline retrosynthesis trees.

Like reference numbers and designations in the various drawings indicate like elements. Features of the example processes can be combined.

### DETAILED DESCRIPTION

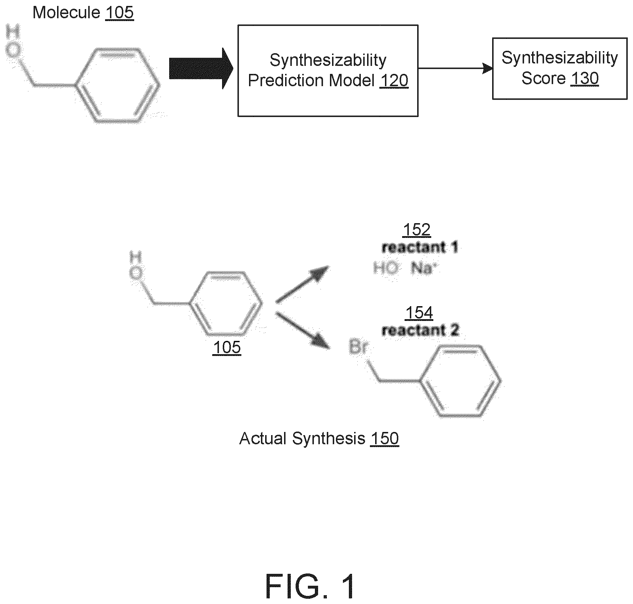

FIG. 1 provides an example overview of predicting a synthesizability score for a molecule. In particular, FIG. 1 depicts how a synthesizability prediction model 120 can provide insight into the actual synthesis of a chemical compound by generating a synthesizability score 130.

In this illustration, the molecule 105 of interest is a benzyl alcohol (C7H8O). For example, the actual synthesis of the molecule 105 can involve a reaction between reactant 1 152, e.g., sodium hydroxide (NaOH), and reactant 2 154, e.g., benzyl bromide. In some cases, the actual synthesis pathway 150 can be provided from a retrosynthesis analysis and validated with a physical experiment. More specifically, a retrosynthesis analysis can be performed, e.g., using a retrosynthesis planner or by programmatically assembling baseline retrosynthesis trees into a new retrosynthesis tree, for identifying an efficient synthesis pathway for a molecule, e.g., originating from basic, commercially available initial reactants.

In this context, a retrosynthesis planner refers to a computational tool configured to generate a retrosynthesis tree for a molecule by decomposing the molecule into simpler reactant molecules. As an example, the retrosynthesis planner can generate a retrosynthesis tree for a molecule by extending an initial retrosynthesis tree using a reaction modeling neural network to iteratively extend one node of the retrosynthesis tree at a time, e.g., as is described in "Generating Retrosynthesis Trees Using Reaction Modeling Neural Networks", U.S. Application No. 63/565,854, and in PCT/EP2024/084567 filed on 3December 2024, which are hereby incorporated by reference in their entirety.

The synthesizability of a target molecule can be an important factor to evaluate when the ability to produce the molecule reliably and cost-effectively is essential, e.g., as part of a drug discovery, materials science, nanotechnology, battery technology, specialty commercial chemicals, etc. process. As depicted, the synthesizability prediction model 120 can replace the need to run a retrosynthesis analysis, e.g., using a retrosynthesis planner, which can be a computationally-intensive process due to the complexity and large number of potential synthesis pathways to evaluate, to determine the ease of synthesizing the molecule based on the potential synthesis pathways generated. Instead, the synthesizability prediction model 120 can process data representing the molecule 105 to generate a synthesizability score 130 directly.

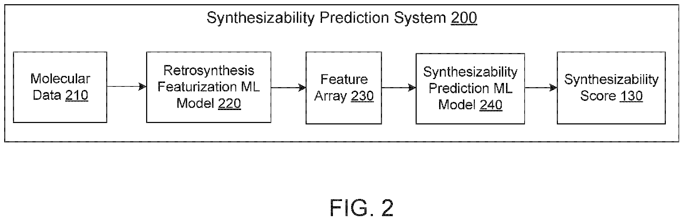

The synthesizability score 130 can characterize the ease of synthesis of a target molecule, e.g., benzyl alcohol 105. In particular, the synthesizability score 130 can conveniently provide a summary value that aggregates a number of factors that are normally evaluated separately for the purposes of measuring synthesizability, e.g., the accessibility and cost of reagents, ease of creating necessary reaction conditions, reaction yield, complexity of synthesis, and the scalability to produce large quantities of the compound. More specifically, the distilled value provided by the synthesizability score 130 can facilitate the use of the molecule in one or more downstream tasks, e.g., as will be described in more detail in FIG. 2.

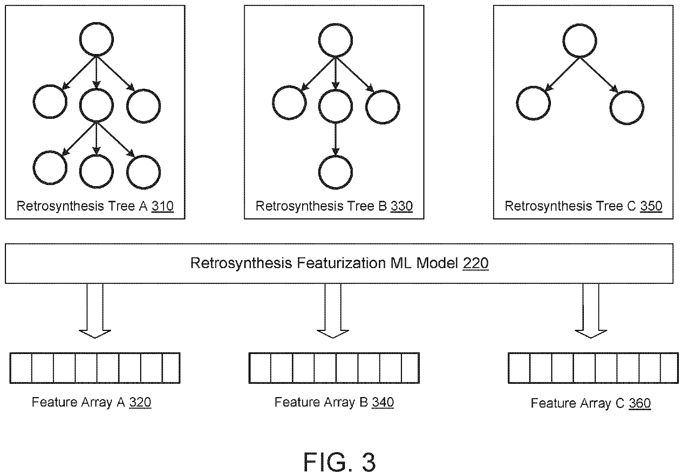

The example of FIG. 1 is simple and is included for illustrative purposes to provide an overview of the synthesizability prediction model 120 generating a synthesizability score 130 that characterizes the synthesis of the molecule 105. While the actual synthesis 150 pathway depicted in FIG. 1 is simple, e.g., the actual synthesis 150 has only two precursors 152 and 154, the synthesizability prediction model 120 can be used to generate a synthesizability score 130 for a molecule with any arbitrary synthesis complexity. In particular, a retrosynthesis tree generated for the molecule can be complex, e.g., have a depth of at least 5, 10, or 15 precursor pathways, and can include any appropriate number of nodes, e.g., at least 10, at least 20, or at least 30 nodes. An example of that includes retrosynthesis trees of an arbitrary complexity will be described in more detail with respect to FIG. 3.

FIG. 2 shows an example synthesizability prediction system 100. The synthesizability prediction system 200 is an example of a system implemented as computer programs on one or more computers in one or more locations in which the systems, components, and techniques described below are implemented.

In particular, the synthesizability prediction system 200 can include a retrosynthesis featurization machine learning model 220 and a synthesizability prediction machine learning model 240 that can be configured to generate a synthesizability score 130 from molecular data 210 in a two-stage process that distills the complexity of predicting a synthesizability score 130.

As an example, the molecular data 210 can include data that characterizes a molecule. For example, the molecular data 210 can include data that represents the chemical structure of a molecule, including data representing one or more of: the atomic composition of the molecule, the types of chemical bonds (e.g., single, double, or triple) between atoms and how these bonds connect the atoms to form the molecule, the spatial arrangement of the atoms (e.g., indicating the three-dimensional (3D) structure of the molecule, the types of functional groups present in the molecule (e.g., hydroxyl groups or carboxyl groups), and so forth. In some cases, the molecular data 210 includes a Simplified Molecular Input Line Entry System (SMILES) string, e.g., which provides a representation of the chemical structure of the molecule in a one-dimensional string. As another example, a representation based on a SELFIES (SELF-referencing Embedded Strings) may be used. In some other cases, the molecular data 210 includes a molecular fingerprint of the molecule. Such a molecular fingerprint can comprise structured data that represents a structure of the molecule in any convenient manner, e.g., dictionary-based, shape-based, topological, and so forth. In other cases, the molecular data 210 includes an International Chemical Identifier (InChI) string defining the chemical structure of the molecule.

The system 100 can process the molecular data 210 using the retrosynthesis featurization machine learning model 220 to generate a feature array 230, e.g., a feature vector including a number of features that characterize a retrosynthesis tree for the molecule. In particular, the feature array 230 can include a set of one or more features characterizing the retrosynthesis tree of the molecule and a set of one or more biophysical and biochemical properties of the molecule.

As one example, the feature array 230 can include a depth, e.g., the longest path from a root node of the retrosynthesis tree to any leaf node of the retrosynthesis tree, a number of nodes, and the probability of the reactions involved in the tree, e.g., as a proxy for the difficulty of synthesizing the molecule via the synthesis pathway represented by the retrosynthesis tree. As another example, the feature array 230 can include a reactivity indicator signifying whether the molecule at the root node is reactive with reference to the retrosynthesis tree, e.g., specifying a speed or degree to which the molecule can be generated by the reactant molecules represented by the leaf nodes. As another example, the feature array 230 can include a stability indicator signifying whether the molecule is unstable with reference to the retrosynthesis tree, e.g., specifying the relative stability of the molecule with respect to one or more other molecules in the retrosynthesis tree. As yet another example, the feature array 230 can include a completeness feature indicative of whether the molecules represented by the leaf nodes in the retrosynthesis tree are included in a predefined library of precursor molecules.

As a further example, the feature array 230 can include the molecular weight, topological polar surface area, formal charge, or number of hydrogen acceptors and donors in the molecule. As yet a further example, the feature array 230 can include the number of rotatable bonds, number of aromatic and aliphatic rings, number of carbon-carbon single bonds, number of heavy atoms, or number of heteroatoms in the molecule. It will be appreciated that all these features can be readily derived from the retrosynthesis tree for the molecule.

The retrosynthesis featurization machine learning model 220 can be implemented as any appropriate type of machine learning model that enables the retrosynthesis featurization machine learning model to perform its described functions. For instance, the retrosynthesis featurization machine learning model can be implemented by a model that includes one or more of: a neural network, or a random forest, or a decision tree, or a support vector machine, and so forth. For instance, the retrosynthesis featurization machine learning model 220 can be implemented as a neural network model that includes appropriate number of neural network layers (e.g., 5 layers, 10 layers, or 15 layers) of any appropriate type (e.g., fully-connected layers, attention layers, convolutional layers, etc.) and connected in any appropriate configuration (e.g., as a linear sequence of layers, or as a directed graph of layers).

In a particular example, the retrosynthesis featurization machine learning model 220 can be implemented as a graph neural network (GNN). In this case, the model input to the retrosynthesis featurization machine learning model 220 can be represented as a graph. As an example, the molecular data 210 received by the system 200 can be a graph, e.g., where the atoms are represented as nodes and chemical bonds are represented as edges. As another example, the system 200 can process the molecular data 210 to generate a graph representation, e.g., where the atoms of the molecule are represented as nodes and the chemical bonds (between the atoms) of the molecule are represented as edges.

In particular, the system 200 can process a set of node features for each atom, e.g., using an encoder block of the GNN, to generate respective node embeddings that represent each atom. The node features for an atom can include features characterizing one or more of: the element type of the atom, the element types of other atoms that are bonded to the atom, the number of hydrogen atoms that are bonded to the atom, whether the atom is included in a functional group (e.g., a carboxyl group), and so forth. The system 200 can additionally process a set of edge features for each chemical bond connecting a pair of atoms, e.g., using the encoder block of the GNN, to generate respective edge embeddings that represent each chemical bond. The edge features for an edge representing a chemical bond can include features characterizing one or more of: the bond type (e.g., single, double, triple, and so forth), which atoms are connected by the bond, whether the bond is part of an aromatic system, and so forth. The system can then use message passing, a method for updating node embeddings based on the aggregation of information from nearby node embeddings into messages, to update the node and edge embeddings with respect to the relationships between them, e.g., based on the chemical bonds and nearby atoms. By aggregating messages from nearby, e.g., neighboring nodes that interact in the molecule, the GNN can implicitly recognize patterns and correlations between the graph structure and associated molecular properties. For example, the system 200 can process the final embeddings of the nodes in the graph using a decoder block of the GNN, to generate the feature array 230.

In more detail, the GNN can include an encoder block, a sequence of one or more message passing neural network layers, and a decoder block. The encoder block can, for each atom in the molecule, process a set of atom features of the atom to generate a node embedding for the graph node that represents the atom. Further, the encoder block can, for each bond in the molecule, process a set of bond features of the bond to generate an edge embedding for the graph edge that represents the edge. Each message passing layer is configured to process the set of node embeddings and the set of edge embeddings, by neural network operations that are parametrized by a set of neural network parameters of the message passing layer and that are conditioned on the topology of the graph representing the molecule, to update the node embeddings and the edge embeddings. The decoder block can process the node, and optionally edge, embeddings generated by the final message passing layer to generate the GNN output, in this case, the feature array characterizing the retrosynthesis tree for the molecule.

The system 200 can train the retrosynthesis featurization machine learning model 220 on a set of training molecules using a machine learning training technique to optimize an objective function. The objective function can measure, for each training example, a discrepancy between: (i) a training target feature array for the training molecule and (ii) a predicted feature array generated by processing the training molecule data using the retrosynthesis featurization machine learning model 220. The objective function can measure a discrepancy between the target and predicted feature array in any appropriate way, e.g., using a cross-entropy loss or a mean squared error loss. The machine learning training technique can be any technique appropriate for training the retrosynthesis featurization machine learning model 220, e.g., by calculating and backpropagating gradients of the objective function to update parameter values of the model 220, e.g., using the update rule of any appropriate gradient descent optimization algorithm, e.g., RMSprop or Adam.

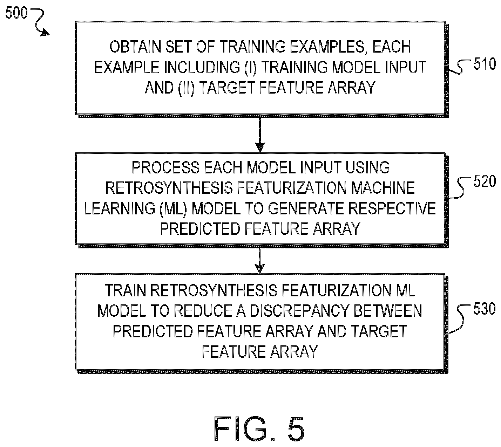

In some cases, the system 200 can generate the target feature arrays for each training molecule in a training example, e.g., by using a retrosynthesis planner to generate one or more retrosynthesis trees for the training molecule, e.g., as will be described in more detail with respect to FIG. 5. In particular, the system 200 can obtain a set of retrosynthesis trees for each of a number of training molecules, e.g., from the retrosynthesis planner, and can generate the target features of the target feature array for each training molecule by processing the one or more retrosynthesis trees.

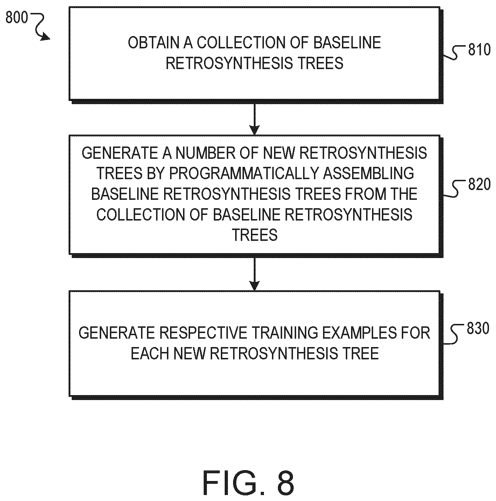

In other cases, the system 200 can generate the target feature arrays by programmatically assembling baseline retrosynthesis trees from a collection of retrosynthesis trees into a new retrosynthesis tree, e.g., as will be described in more detail with respect to FIG. 8. In this case, the system 200 can generate a respective training example for each new retrosynthesis tree, e.g., the training example can include a training molecule represented by the root node of the retrosynthesis tree, and the system can generate the target features of the target feature array for the training molecule by processing the one or more retrosynthesis trees.

More specifically, the system 200 can process each of the one or more predicted retrosynthesis trees to generate a corresponding feature array and, in the case that there is more than one predicted retrosynthesis tree, can aggregate, e.g., sum, average, etc., the feature arrays generated for each of the retrosynthesis trees to generate the target feature array for the training molecule. In general, a feature array, i.e. the target feature array, can be derived from a predicted retrosynthesis tree in a similar way to that previously described.

For example, the system 200 can identify the depth of the (predicted) retrosynthesis tree and the number of nodes of the retrosynthesis tree as target features. As another example, the system 200 can compute the probability of the reaction as a target feature, e.g., by generating the probability of each of the predicted reactions of the retrosynthesis tree and summing the log-probabilities.

As an example, the system 200 can receive the probability of one or more of each predicted reaction in the tree, e.g., from a retrosynthesis planner that is configured to generate the likelihood of the reaction between the molecules of the child nodes of each non-leaf node that would generate the molecule represented by the leaf node. There are many examples of retrosynthesis planners; merely as an example the previously indicated retrosynthesis planner can be used.

Also or instead the system 200 can use an additional machine learning model, e.g., a reaction likelihood machine learning model, to generate the likelihood of each reaction by processing data relating to a subset of the retrosynthesis tree. For instance, the reaction likelihood machine learning model can be implemented as a neural network model that includes any appropriate number of neural network layers (e.g., 5 layers, 10 layers, or 15 layers) of any appropriate type (e.g., fully-connected layers, attention layers, convolutional layers, etc.) and connected in any appropriate configuration (e.g., as a linear sequence of layers, or as a directed graph of layers). As an example, the reaction likelihood machine learning model can be a generative model, e.g., the reaction likelihood machine learning model can have a recurrent neural network architecture that is configured to sequentially process the contents of an input and that is trained to perform next element prediction, e.g., to define a likelihood score distribution over a set of next elements. A likelihood for a sequence of predicted elements can be obtained from a product of the likelihood of each element, or by summing the log likelihood of each predicted element. As some examples, the reaction likelihood machine learning model can include a recurrent neural network (RNN), long short-term memory (LSTM), gated-recurrent unit (GRU), or an encoder-decoder transformer.

In particular, the system 200 can process an input that defines a collection of molecules using the reaction likelihood machine learning model to generate an output that defines a likelihood of a reaction between the molecules in the collection. As an example, the reaction likelihood machine learning model can have been configured to process an ordered list including SMILES string or molecular fingerprint representations of the product molecule of a reaction followed by any precursor molecules that are involved in the synthesis pathway to generate a value representing a probability of the reaction occurring.

As another example, the system 200 can determine the stability of the molecule with reference to the retrosynthesis tree as a target feature by analyzing the structure of the molecule and the intermediate reactants in the retrosynthesis tree. In some cases, the system 200 can identify functional groups, or structural features that can lead to strain, steric hindrance, or resonance stabilization (e.g., from aromatic rings) in the molecule, and any unstable intermediate reactant molecules in the retrosynthesis tree for the molecule, e.g., based on the presence of carbenes, radicals, or highly-strained bonds in the molecules, to determine whether the molecule is stable. The system 200 can also evaluate the heat of formation and other chemical conditions required for synthesis to determine stability, e.g., since molecules that require a high heat or extreme conditions for formation are generally less stable.

As yet another example, the system 200 can determine the reactivity of the molecule with reference to the retrosynthesis tree as a target feature, e.g., by identifying intermediate reactant molecules that are prone to spontaneous reaction, e.g., based on the presence of leaving groups, e.g., a group of atoms that tend to detach from a molecule during a chemical reaction, or electrophilic centers. The system 200 can also evaluate whether the molecule is known to decompose experimentally, or if the molecule is sensitive to air, light, or moisture to determine reactivity.

As a further example, the system 200 can determine the completeness of the retrosynthesis tree by evaluating whether the molecules represented by the leaf nodes in the retrosynthesis tree are in a predefined library of precursor molecules.

The system 200 can then process the feature array 230 using a synthesizability prediction machine learning model 240 to generate the synthesizability score 130. The synthesizability score 130 can characterize the ease of synthesis of a target molecule, e.g., based on one or more of: the complexity and number of steps required to synthesize the molecule (including the types of chemical reactions involved and their respective yields), the scalability of the synthesis process (e.g., how readily the synthesis process can be scaled from laboratory scale production to industrial scale production), the stability of intermediate compounds throughout the synthesis process, the reaction yield of the synthesis process, the reaction conditions required to carry out the synthesis process, and so forth.

The synthesizability prediction machine learning model 240 can be implemented as any appropriate type of machine learning model that enables the synthesizability prediction machine learning model to perform its described functions. For instance, the synthesizability prediction machine learning model can be implemented by a model that includes one or more of: a neural network, or a random forest, or a decision tree, or a support vector machine, and so forth. For instance, the synthesizability prediction machine learning model 240 can be implemented as a neural network model that includes appropriate number of neural network layers (e.g., 5 layers, 10 layers, or 15 layers) of any appropriate type (e.g., fully-connected layers, attention layers, convolutional layers, etc.) and connected in any appropriate configuration (e.g., as a linear sequence of layers, or as a directed graph of layers).

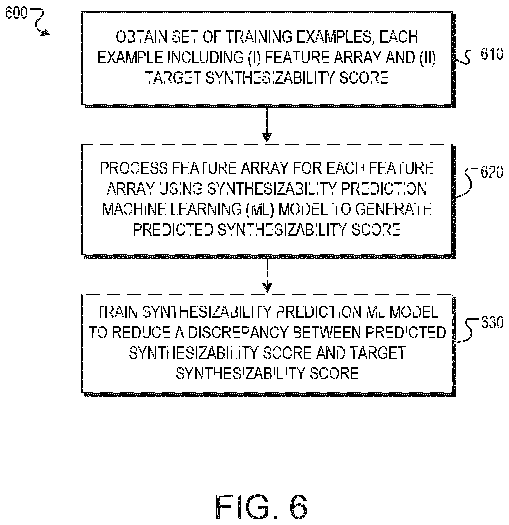

The system 200 can train the synthesizability prediction machine learning model 240 on a set of training feature arrays using a machine learning training technique to optimize an objective function. The objective function can measure, for each training example, a discrepancy between: (i) a training target synthesizability score for the training molecule and (ii) a predicted synthesizability score generated by processing the training feature array using the synthesizability prediction machine learning model 240. An example process for training the synthesizability prediction machine learning model will be described in more detail with reference to FIG. 5. In the case that the synthesizability prediction machine learning model 240 is implemented as a differentiable model, e.g., a neural network, the system 200 can jointly train both the retrosynthesis featurization machine learning model 220 and the synthesizability prediction machine learning model 240. An example process for jointly training the retrosynthesis featurization machine learning model and the synthesizability prediction machine learning model will be covered in more detail with reference to FIG. 6.

The synthesizability score 130 can be a value, e.g., as determined from a regression, or a label, e.g., as determined from a classification. In the case that the synthesizability score 130 is a value, the score 130 can be a value determined within a range of allowable synthesizability scores, e.g., from 0-100, 0-1000, etc., e.g., to retain relative meaning between predicted synthesizability scores. In the case that the synthesizability score 130 is a label, the synthesizability score can be determined from a set of labels indicating a difficulty of synthesis, e.g., "easy", "basic", "intermediate", "challenging", or "complex".

In some cases, the system 200 can provide the synthesizability score 130 for one or more downstream tasks. For example, the synthesizability score 130 can be used to inform decisions that require selecting one or more molecules from a collection of molecules. In particular, the system can receive a collection of candidate molecules, can generate the synthesizability for each of the candidate molecules, and can select one or more of the candidate molecules for physical synthesis. More specifically, the system can be used to select molecules for further evaluation as part of drug discovery and to identify molecules for inclusion in a virtual screening library. In some cases, after selection, the candidate molecules can be physically synthesized and physical experiments can be performed to further evaluate the candidate molecule, e.g., to determine one or more properties of the physically synthesized molecule.

As another example, synthesizability scores generated by the synthesizability prediction system can be used to perform classifier guidance for a generative machine learning model that generates data defining molecules, in particular, to encourage the generative machine learning model to generate molecules having "high" synthesizability scores, e.g., that satisfy (e.g., exceed) a threshold. In more detail, classifier guidance is a technique employed to improve the output quality of generative machine learning models by integrating a "classifier" (in this case, the synthesizability prediction system) to influence the generation process. Classifier guidance can be applied across a wide variety of generative machine learning models, e.g., generative diffusion models, generative adversarial networks, autoregressive language models, and so forth.

In particular, the synthesizability prediction system can be used to evaluate and guide the output molecules generated by the generative model, e.g., as the generative model produces candidate output molecules which are then assessed for synthesizability by the synthesizability prediction system. That is, the synthesizability prediction system can process data defining candidate output molecules generated by the generative model to generate corresponding synthesizability scores. Based on the synthesizability scores, the generative model adjusts its output generation process in order to generate output molecules having higher synthesizability scores.

The synthesizability scores generated by the synthesizability prediction system for output molecules generated by the generative model can be used to guide the generative model in any of a variety of possible ways.

For instance, a generative system (that includes the generative model) can perform rejection sampling, where output molecules with synthesizability scores that fail to satisfy a threshold are discarded and new output molecules are generated until one or more output molecules with synthesizability scores that satisfy the threshold are generated.

As another example, a generative system can perform classifier guidance using the synthesizability scores generated by the synthesizability prediction system, e.g., by performing gradient-based adjustment of intermediate outputs generated by the generative model. For instance, the generative model may be a generative diffusion model that generates an output molecule by iteratively denoising the 3D spatial positions of the atoms in the molecule over a sequence of denoising iterations using a denoising neural network. At each of one or more denoising iterations, the generative diffusion model can generate a partially denoised output molecule which is then processed by the synthesizability prediction system to generate a corresponding synthesizability score. Gradients of the synthesizability score are then computed with respect to parameters defining the 3D spatial positions of the atoms in the partially denoised output molecule, e.g., by backpropagation. The gradients are then used to adjust the 3D spatial positions of the atoms in the molecule to encourage an increase in the synthesizability score of the molecule, e.g., using the update rule of an appropriate gradient descent optimization algorithm, e.g., RMSprop or Adam. The generative diffusion model can then proceed to the next denoising iteration using molecule that has been modified using the gradients of the synthesizability score.

As another example, a generative system can use the synthesizability scores generated by synthesizability prediction system as a reward for output molecules generated by a generative model, and then train the generative model by a reinforcement learning technique to encourage an increase in the synthesizability scores of output molecules generated by the generative model.

In some cases, the generative machine learning model can be configured to generate output ligand molecules that are predicted to bind to a target protein molecule, e.g., to achieve a therapeutic effect in a patient. For example, the target protein molecule can be an agonist or antagonist of a receptor or enzyme. In this example, the performing classifier guidance using the synthesizability prediction system can encourage the generative machine learning model to generate output molecules that both: (i) bind to the target protein molecule, and (ii) have high synthesizability, e.g., as characterized by a synthesizability score that exceeds a threshold.

As described above, the synthesizability score 130 can be used as an input to a machine learning model as a measure of synthetic accessibility. In particular, the synthesizability score 130 can be used to perform classifier guidance for a generative model, e.g., a generative diffusion model that is configured to generate a ligand molecule for binding to a target protein. In this case, the synthesizability score 130 can be used to encourage the generation of ligands with a threshold level of synthesizability. More specifically, classifier guidance is a technique for guiding the output of generative diffusion models to conform to desired characteristics, e.g., based on a particular class or specific attributes. In this context, the system can provide synthesizability scores to a generative diffusion model at each of a number of denoising iterations, e.g., to ensure that the generated ligand molecules for binding to the target protein are steered toward a certain (defined) level or threshold of synthesizability, and towards ligand molecules that are relatively easier to synthesize. For example, the system can define an objective function using the synthesizability score of the generated ligands and can train the generative machine learning model by calculating and backpropagating gradients of the objective function to update the parameter values of the generative diffusion model, e.g., using the update rule of any appropriate gradient descent optimization algorithm, e.g., RMSprop or Adam.

In particular, in applications such as using synthesizability scores to perform classifier guidance, which can require generating a large number of synthesizability scores, e.g., at each of a large number of denoising iterations when generating molecules using a generative diffusion model, this can enable a significant reduction of computational resources with a corresponding gain in computational efficiency.

FIG. 3 depicts an example of processing retrosynthesis trees to generate corresponding feature arrays using the retrosynthesis featurization machine learning model 220 of FIG. 2.

In particular, FIG. 3 depicts three retrosynthesis trees, e.g., retrosynthesis tree A 310, retrosynthesis tree B 330, and retrosynthesis tree C 350. The retrosynthesis trees represent a respective reactant molecule involved in a chemical reaction that produces the molecule represented by the non-leaf node. More specifically, each tree has a root node representing a molecule and each child node of the root node represents a reactant of a pathway to synthesis the parent node.

In particular, a retrosynthesis tree generated for a molecule can be complex, e.g., have a depth of at least 5, 10, or 15 precursor pathways, and can include any appropriate number of nodes, e.g., at least 10, at least 20, or at least 30 nodes. Retrosynthesis trees with greater depth are more complex, e.g., in this case, tree A 310 and tree B 330 have three levels, and tree C has two levels. Despite the variance in retrosynthesis tree structure, the system can process data characterizing the respective molecule corresponding to each retrosynthesis tree, e.g., retrosynthesis tree A 310, retrosynthesis tree B 330, and retrosynthesis tree C 350, using the retrosynthesis featurization model 220 to generate the respective feature array A 320, feature array B 340, and feature array 360.

As described with respect to FIG. 2, the feature arrays 320, 340, and 360 can include a set of features characterizing the retrosynthesis tree and a set of biophysical and biochemical properties of the molecule. For example, the set of features characterizing the retrosynthesis tree can characterize the difficulty of synthesis based on the structure of the retrosynthesis tree, e.g., the depth, number of nodes, and probability of the reaction involved. The set of features characterizing the retrosynthesis tree can also include a Boolean feature that indicates whether the molecule is reactive or not with reference to the retrosynthesis tree, a Boolean feature that indicates whether the molecule is unstable or not with reference to the retrosynthesis tree, and a Boolean feature that indicates whether the retrosynthesis tree is complete, e.g., whether the intermediate reactants in the retrosynthesis tree are in a predefined library of precursor molecules that are easily obtainable for synthesis.

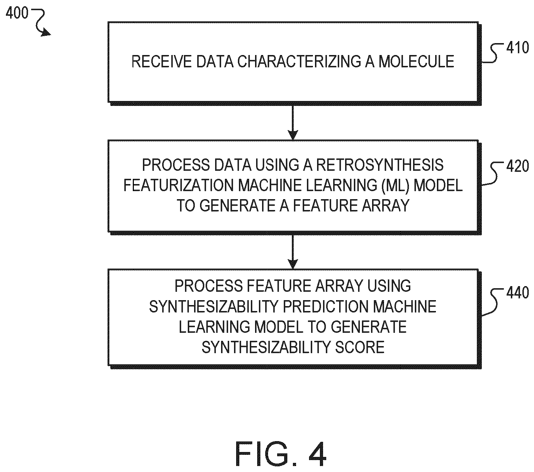

FIG. 4 is a flow diagram of an example process 400 for generating a synthesizability score. For convenience, the process 300 will be described as being performed by a system of one or more computers located in one or more locations. For example, a synthesizability prediction system, e.g., the synthesizability prediction system 200 of FIG. 2, appropriately programmed in accordance with this specification, can perform the process 400.

The system receives data characterizing a molecule (step 410). In particular, the system can receive data including data defining a chemical structure of the molecule. As an example, the system can receive a Simplified Molecular Input Line Entry System (SMILES) string that represents the structure of the molecule. As another example, the system can receive a molecular fingerprint that represents the structure of the molecule. As yet another example, the system can receive a structural data file, e.g., a protein databank file, structure data file, chemical markup file, etc. The system can receive the data characterizing the molecule, e.g., by way of a user interface (e.g., a graphical user interface, GUI) or application programming interface (API) made available by the user, e.g., from a user or from a separate upstream system.

The system processes the data characterizing the molecule using a retrosynthesis featurization machine learning model to generate a feature array (step 420). The feature array can include a set of one or more features characterizing the retrosynthesis tree and a set of one or more biophysical and biochemical properties of the molecule. In particular, the retrosynthesis featurization machine learning model can be a neural network that has been trained to process data characterizing a molecule to generate a feature array. An example process for training a retrosynthesis featurization machine learning model will be covered in more detail with reference to FIG. 4. In some implementations, the retrosynthesis featurization machine learning model is implemented as a graph neural network, as described above with reference to FIG. 2.

The system processes the feature array using the synthesizability prediction machine learning model to generate the synthesizability score (step 430). The synthesizability score can characterize the ease of synthesis of a target molecule, e.g., based on one or more of the accessibility and cost of reagents, ease of creating necessary reaction conditions, reaction yield, complexity of synthesis, and the scalability to produce large quantities of the compound. Such a characterization of the ease of synthesis of the target molecule is also referred to herein as the synthesis complexity of the target molecule. As an example, the synthesizability prediction machine learning model can be a random forest model, decision tree model, support vector machine model, linear regression model, etc. As another example, neural network that has been trained to process a feature array to generate a synthesizability score. An example process for training a synthesizability machine learning model will be described in more detail with reference to FIG. 6.

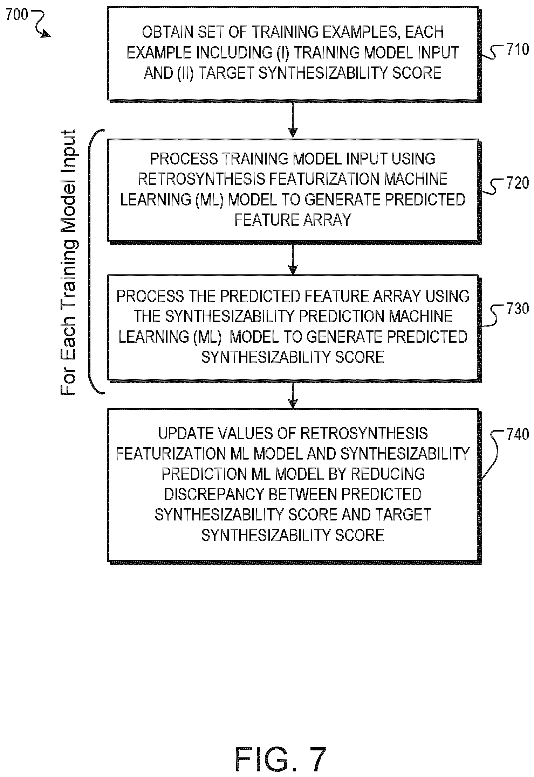

In the case that the synthesizability prediction machine learning model is a differentiable machine learning model, the system can jointly train the retrosynthesis featurization machine learning model and the synthesizability prediction machine learning model. An example process for jointly training the retrosynthesis featurization machine learning model and the synthesizability prediction machine learning model will be described in more detail with reference to FIG. 7.

The system can provide the synthesizability score, e.g., for storage in a memory, or for presentation on a display of a user device, or for transmission across a data communications network, or for further processing by a downstream system, e.g., for performing classifier guidance of generative machine learning model, or for filtering a set of candidate molecules to select one or more molecules for physical synthesis.

FIG. 5 is a flow diagram of an example process 500 for training the retrosynthesis featurization machine learning model. For convenience, the process 500 will be described as being performed by a system of one or more computers located in one or more locations. For example, a synthesizability prediction system, e.g., the synthesizability prediction system 200 of FIG. 2, appropriately programmed in accordance with this specification, can perform the process 500.

The system can obtain a set of training examples, where each training example corresponds to a respective training molecule and includes: (i) a training model input, and (ii) a target feature array (step 510). In particular, the training model input can include data that characterizes the training molecule, e.g., data including data defining a chemical structure of the training molecule, e.g., a SMILES string, and the target feature array can include a set of features characterizing a retrosynthesis tree for the training molecule.

The system can generate the target feature array for a training example by obtaining a retrosynthesis tree for the training molecule, and then processing the retrosynthesis tree to obtain a set of features of the retrosynthesis tree that collectively define the target feature array. For example, the system can process data characterizing the training molecule using a retrosynthesis planner to generate a number of retrosynthesis trees for the training molecule, e.g., one retrosynthesis tree or many, e.g., 5, 10, 20, retrosynthesis trees. As an example, the retrosynthesis planner can be implemented as a machine learning model having a set of machine learning model parameters that are trained by a machine learning training technique.

The system can process one or more of the predicted retrosynthesis trees for the training molecule to generate the target feature array of the training example. The target feature array for a training example can include any appropriate number of retrosynthesis tree features, e.g., 5 features, or 10 features, or 100 features. In particular, the target feature array can include a set of features characterizing the retrosynthesis tree and a set of biophysical and biochemical properties of the molecule.

For example, the set of one or more features characterizing the retrosynthesis tree can include a predicted depth, number of nodes, and probability of reactions involved. As another example, the set of one or more features characterizing the retrosynthesis tree can include a reactivity indicator signifying whether the molecule is reactive with reference to the retrosynthesis tree and a stability indicator signifying whether the molecule is unstable with reference to the retrosynthesis tree. As yet another example, the set of one or more features characterizing the retrosynthesis tree can include a completeness indicative of whether the intermediate reactants represented by the nodes in the retrosynthesis tree are obtainable for synthesis. As a further example, the set of one or more features characterizing the retrosynthesis tree can include one or more biophysical and biochemical properties of the molecule.

As an example, the system can generate features in the target feature array by analyzing the structure of the retrosynthesis tree, e.g., to identify the depth, number of nodes, etc. As another example, the system can determine the stability and reactivity of a molecule as features by analyzing the structure of the molecule and the intermediate reactants in the retrosynthesis tree. As yet another example, the system can determine whether the intermediate reactants represented by the nodes in the retrosynthesis tree are in a predefined library of precursor molecules that are obtainable for synthesis.

More specifically, the system can process each of the one or more predicted retrosynthesis trees to generate a corresponding feature array and, in the case that there is more than one predicted retrosynthesis tree, can aggregate, e.g., sum, average, etc., the feature arrays generated for each of the retrosynthesis trees to generate the target feature array. In some cases, the system can select a set of at least one, but fewer than all of the predicted retrosynthesis trees to generate the target feature arrays, e.g., before aggregating. In particular, the system can rank the predicted retrosynthesis trees based on one or more criteria to select the set or can cluster the trees to select the set based at least on a measure of diversity of synthesis (diversity can be measured, e.g., by counting reactant molecules that are different or shared). As an example, the system can rank the predicted retrosynthesis trees based on their depth, total number or nodes, total number of subtrees with more than one child node, etc. In some cases, the system can select the simplest trees from the ranking, e.g., one or more trees having the smallest values of the ranking criteria.

In some cases, for one or more of the training examples, the system can obtain the retrosynthesis tree for the training molecule by automatically parsing a body of scientific literature (e.g., academic literature) using natural language processing techniques. In other cases, the system can generate the retrosynthesis tree of the training examples. An example for generating a new retrosynthesis tree by programmatically assembling baseline retrosynthesis trees and generating the target feature array for the generated retrosynthesis tree as a training example using the new retrosynthesis tree will be described in more detail with respect to FIG. 8.

The system can process the model input using a retrosynthesis featurization machine learning model to generate a predicted feature array (step 520), e.g., for each of the training model inputs in the set of training examples. In particular, the retrosynthesis featurization machine learning model can be a neural network, e.g., a graph neural network as described in FIG. 4, that is configured to process the model input to generate features characterizing the retrosynthesis tree of the molecule and one or more biochemical or biophysical properties as the feature array. For example, the properties can include the molecular weight, topological polar surface area, or formal charge. As another example, the properties can include the number of hydron acceptors and donors, number of rotatable bonds, number of aromatic and aliphatic rings, number of carbon-carbon single bonds, number of heavy atoms, or number of heteroatoms properties.

The system can then train the retrosynthesis featurization machine learning model to reduce a discrepancy between the predicted feature array and the target feature array (step 530). In particular, the discrepancy can be included in an objective function as a loss, e.g., a cross-entropy or mean-squared error loss. For example, the retrosynthesis featurization machine learning model can be trained by calculating and backpropagating gradients of the objective function to update parameter values of the model, e.g., using the update rule of any appropriate gradient descent optimization algorithm, e.g., RMSprop or Adam, to minimize the loss over a number of training iterations.

FIG. 6 is a flow diagram of an example process 600 for generating for training the synthesizability machine learning model. For convenience, the process 600 will be described as being performed by a system of one or more computers located in one or more locations. For example, a synthesizability prediction system, e.g., the synthesizability prediction system 200 of FIG. 2, appropriately programmed in accordance with this specification, can perform the process 600.

The system can obtain a set of training examples, each example including (i) a feature array and (ii) a target synthesizability score (step 610). In particular, the feature array can include a set of one or more features characterizing the retrosynthesis tree of the molecule and a set of one or more biophysical and biochemical properties of the molecule, and the target synthesizability score can be a ground truth value or class label indicating the ease of synthesis of the target molecule represented by the feature array. In some cases, the system can have generated the feature array by processing a model input including data characterizing a molecule with a retrosynthesis featurization machine learning model that has been trained, e.g., using the process 500. In other cases, the feature arrays can be obtained from known retrosynthesis trees, e.g., the system can implement natural language processing techniques to scrape, e.g., extract the data of, retrosynthesis trees from scientific literature databases, repositories, or both. In some cases, given a collection of retrosynthesis trees that are obtained, e.g., through computational generation or from literature database or repository, the system can generate new retrosynthesis trees by combining/stacking multiple retrosynthesis trees into a single combined tree.

The system can obtain the target synthesizability scores in any of a variety of possible ways. For instance, the target synthesizability scores can be generated by manual annotation and labeling by experts, e.g., chemists. As another example, the target synthesizability scores can be generated by an automated process, e.g., where each target synthesizability score for a molecule is generated as a function (e.g., a linear combination) of respective numerical values characterizing one or more of: the complexity and number of steps required to synthesize the molecule, the scalability of the synthesis process, the stability of intermediate compounds throughout the synthesis process, the reaction yield of the synthesis process, the reaction conditions required to carry out the synthesis process, and so forth.

The system can process the feature array for each training example using the synthesizability prediction machine learning model to generate a predicted synthesizability score (step 620), e.g., for each feature array in the set of training examples. In particular, the synthesizability prediction machine learning model can be configured to process the feature array to generate a predicted synthesizability score, e.g., a predicted value or predicted class label. As an example, the synthesizability prediction machine learning model can be a random forest model, decision tree model, support vector machine model, linear regression model, etc. As another example, the synthesizability prediction machine learning model can be a neural network.

The system can then train the synthesizability prediction machine learning model to reduce a discrepancy between the predicted synthesizability score and target synthesizability score (step 630). For example, in the case that the synthesizability prediction machine learning model is a support vector machine, the system can use a convex optimization technique, e.g., sequential minimal optimization, kernel trick, regularization parameter optimization, to find a hyperplane that best fits the training data, e.g., minimizes the discrepancy between the predicted synthesizability score and the target synthesizability score.

In the case that the synthesizability prediction machine learning model is differentiable, e.g., a neural network, the discrepancy can be included in an objective function as a loss, e.g., a cross-entropy or mean-squared error loss. In this case, the synthesizability prediction machine learning model can be trained by calculating and backpropagating gradients of the objective function to update parameter values of the model, e.g., using the update rule of any appropriate gradient descent optimization algorithm, e.g., RMSprop or Adam, to minimize the loss over a number of training iterations. In particular, in the case that the synthesizability prediction machine learning model is differentiable, the system can jointly train the retrosynthesis featurization machine learning model and the synthesizability machine learning models, as described in FIG. 7.

FIG. 7 is a flow diagram of an example process 700 for jointly training the retrosynthesis featurization and synthesizability machine learning models. For convenience, the process 700 will be described as being performed by a system of one or more computers located in one or more locations. For example, a synthesizability prediction system, e.g., the synthesizability prediction system 200 of FIG. 2, appropriately programmed in accordance with this specification, can perform the process 700.

The system can obtain a set of training examples, each example including (i) a training model input and (ii) a target synthesizability score (step 710). In particular, the training model input can include data that characterizes a training molecule, e.g., data including data defining a chemical structure of the molecule, e.g., a SMILES string, and the target synthesizability score can be a ground truth value or class label indicating the ease of synthesis of the target molecule represented by the feature array.

The system can process the training model input using the retrosynthesis featurization machine learning model to generate a predicted feature array (step 720), and the system can process the predicted feature array using the synthesizability prediction machine learning mode to generate the predicted synthesizability score (step 730). In this case, the system can chain the processing of the two-step model to generate the predicted synthesizability score as the final output for each of the training model inputs.

The system can then update values of the retrosynthesis featurization machine learning model and the synthesizability prediction machine learning model by reducing the discrepancy between the predicted synthesizability score and target synthesizability score (step 740). More specifically, the discrepancy between the predicted synthesizability score and the target synthesizability score can be included a joint objective function as a loss, e.g., a cross-entropy or mean-squared error loss. The system can then train both models by calculating and backpropagating gradients of the objective function to update parameter values of both the retrosynthesis featurization machine learning model and the synthesizability prediction machine learning model. For example, the system can use the update rule of any appropriate gradient descent optimization algorithm, e.g., RMSprop or Adam, to minimize the loss over a number of training iterations.

FIG. 8 is a flow diagram of an example process for generating training examples by programmatically assembling baseline retrosynthesis trees. For convenience, the process 800 will be described as being performed by a system of one or more computers located in one or more locations. For example, a synthesizability prediction system, e.g., the synthesizability prediction system 200 of FIG. 2, appropriately programmed in accordance with this specification, can perform the process 800.

The system can obtain a collection of baseline retrosynthesis trees (step 810). For example, the system can identify the collection of baseline retrosynthesis trees by scraping a corpus of data using natural language processing techniques, e.g. as previously described. As another example, baseline retrosynthesis trees can be obtained from one of the many open source datasets available. In some implementations, the baseline retrosynthesis trees are used to generate training examples, as described below.

The system can generate a number of new retrosynthesis trees by programmatically assembling baseline retrosynthesis trees from the collection of baseline retrosynthesis trees (step 820). In particular, the baseline retrosynthesis trees can include retrosynthesis trees for one-step reactions, e.g., including a product decomposing into two or more reactants. For example, the system can identify a first baseline retrosynthesis tree including a root node and one or more leaf nodes and can select one or more second baseline retrosynthesis trees with root nodes representing the same molecule as a corresponding leaf node of the first baseline retrosynthesis tree.

More specifically, the system can search a space of possible combinations of baseline retrosynthesis trees having leaf nodes that each represent a molecule included in a predefined library of precursor molecules to identify the new retrosynthesis tree, e.g., the system can search the space of possible complete retrosynthesis trees including the baseline retrosynthesis trees. More specifically, the system can identify a first baseline retrosynthesis tree including a root node and one or more leaf nodes, and can identify a number of second baseline retrosynthesis trees having root nodes that each represent a same molecule as a corresponding leaf node of the first baseline retrosynthesis tree.

In particular, the system can leverage the parent-child node structure to efficiently build a new retrosynthesis tree using dynamic programming, e.g., by breaking down the generation of each new retrosynthesis tree into subcomponents involving identifying a next baseline retrosynthesis tree for each leaf node of the previous retrosynthesis tree. More specifically, the system can recursively select additional baseline retrosynthesis trees with root nodes representing the same molecule as a corresponding leaf node of the previous retrosynthesis tree.

The system can perform the search to optimize an objective function that measures a penalty associated with the new retrosynthesis tree, e.g., the system can select a second baseline retrosynthesis tree for attachment to the first baseline retrosynthesis tree at each of the corresponding leaf nodes based on the penalties. Within the context of dynamic programming, the system can maximize an optimality criterion for the selection of the additional baseline retrosynthesis trees for attachment to the leaves of the previous retrosynthesis tree, e.g., by minimizing the penalty (objective) function.

In particular, the system can determine, for each of the number of second baseline retrosynthesis trees, a penalty that would result from attaching the second baseline retrosynthesis tree to the first baseline retrosynthesis tree at the corresponding leaf node of the first baseline retrosynthesis tree, and can select the second baseline retrosynthesis tree, e.g., with the lowest penalty. As an example, the penalty can characterize one or more of a depth of the new retrosynthesis tree, a measure of ease of procurement of the molecules represented by the nodes in the new retrosynthesis tree, or a yield of the reactions in a synthesis pathway represented by the new retrosynthesis tree.

The system can generate a respective training example for each new retrosynthesis tree (step 830). In particular, the system can generate a training model input for the training example based on a training molecule represented by a root node of the retrosynthesis tree, and generating a target feature array for the training example by processing the new retrosynthesis tree to determine a number of features of the retrosynthesis tree for the training molecule. In particular, the target feature array can include a set of features characterizing the retrosynthesis tree and a set of biophysical and biochemical properties of the molecule, e.g., a number of nodes, probability of reactions involved, a reactivity, etc.

For example, the system can generate features in the target feature array by analyzing the structure of the retrosynthesis tree, e.g., to identify the depth, number of nodes, etc. As another example, the system can determine the stability and reactivity of a molecule as features by analyzing the structure of the molecule and the intermediate reactants in the retrosynthesis tree. As yet another example, the system can determine whether the intermediate reactants represented by the nodes in the retrosynthesis tree are in a predefined library of precursor molecules that are obtainable for synthesis.

This specification uses the term "configured" in connection with systems and computer program components. For a system of one or more computers to be configured to perform particular operations or actions means that the system has installed on it software, firmware, hardware, or a combination of them that in operation cause the system to perform the operations or actions. For one or more computer programs to be configured to perform particular operations or actions means that the one or more programs include instructions that, when executed by data processing apparatus, cause the apparatus to perform the operations or actions.

Embodiments of the subject matter and the functional operations described in this specification can be implemented in digital electronic circuitry, in tangibly-embodied computer software or firmware, in computer hardware, including the structures disclosed in this specification and their structural equivalents, or in combinations of one or more of them. Embodiments of the subject matter described in this specification can be implemented as one or more computer programs, i.e., one or more modules of computer program instructions encoded on a tangible non-transitory storage medium for execution by, or to control the operation of, data processing apparatus. The computer storage medium can be a machine-readable storage device, a machine-readable storage substrate, a random or serial access memory device, or a combination of one or more of them. Alternatively or in addition, the program instructions can be encoded on an artificially-generated propagated signal, e.g., a machine-generated electrical, optical, or electromagnetic signal, that is generated to encode information for transmission to suitable receiver apparatus for execution by a data processing apparatus.

The term "data processing apparatus" refers to data processing hardware and encompasses all kinds of apparatus, devices, and machines for processing data, including by way of example a programmable processor, a computer, or multiple processors or computers. The apparatus can also be, or further include, special purpose logic circuitry, e.g., an FPGA (field programmable gate array) or an ASIC (application-specific integrated circuit). The apparatus can optionally include, in addition to hardware, code that creates an execution environment for computer programs, e.g., code that constitutes processor firmware, a protocol stack, a database management system, an operating system, or a combination of one or more of them.

A computer program, which may also be referred to or described as a program, software, a software application, an app, a module, a software module, a script, or code, can be written in any form of programming language, including compiled or interpreted languages, or declarative or procedural languages; and it can be deployed in any form, including as a stand-alone program or as a module, component, subroutine, or other unit suitable for use in a computing environment. A program may, but need not, correspond to a file in a file system. A program can be stored in a portion of a file that holds other programs or data, e.g., one or more scripts stored in a markup language document, in a single file dedicated to the program in question, or in multiple coordinated files, e.g., files that store one or more modules, sub-programs, or portions of code. A computer program can be deployed to be executed on one computer or on multiple computers that are located at one site or distributed across multiple sites and interconnected by a data communication network.

In this specification the term "engine" is used broadly to refer to a software-based system, subsystem, or process that is programmed to perform one or more specific functions. Generally, an engine will be implemented as one or more software modules or components, installed on one or more computers in one or more locations. In some cases, one or more computers will be dedicated to a particular engine; in other cases, multiple engines can be installed and running on the same computer or computers.

The processes and logic flows described in this specification can be performed by one or more programmable computers executing one or more computer programs to perform functions by operating on input data and generating output. The processes and logic flows can also be performed by special purpose logic circuitry, e.g., an FPGA or an ASIC, or by a combination of special purpose logic circuitry and one or more programmed computers.

Computers suitable for the execution of a computer program can be based on general or special purpose microprocessors or both, or any other kind of central processing unit. Generally, a central processing unit will receive instructions and data from a read-only memory or a random access memory or both. The essential elements of a computer are a central processing unit for performing or executing instructions and one or more memory devices for storing instructions and data. The central processing unit and the memory can be supplemented by, or incorporated in, special purpose logic circuitry. Generally, a computer will also include, or be operatively coupled to receive data from or transfer data to, or both, one or more mass storage devices for storing data, e.g., magnetic, magneto-optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device, e.g., a universal serial bus (USB) flash drive, to name just a few.

Computer-readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks.

To provide for interaction with a user, embodiments of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's device in response to requests received from the web browser. Also, a computer can interact with a user by sending text messages or other forms of message to a personal device, e.g., a smartphone that is running a messaging application, and receiving responsive messages from the user in return.

Data processing apparatus for implementing machine learning models can also include, for example, special-purpose hardware accelerator units for processing common and compute-intensive parts of machine learning training or production, i.e., inference, workloads.

Machine learning models can be implemented and deployed using a machine learning framework, e.g., a TensorFlow framework, or a Jax framework.

Embodiments of the subject matter described in this specification can be implemented in a computing system that includes a back-end component, e.g., as a data server, or that includes a middleware component, e.g., an application server, or that includes a front-end component, e.g., a client computer having a graphical user interface, a web browser, or an app through which a user can interact with an implementation of the subject matter described in this specification, or any combination of one or more such back-end, middleware, or front-end components. The components of the system can be interconnected by any form or medium of digital data communication, e.g., a communication network. Examples of communication networks include a local area network (LAN) and a wide area network (WAN), e.g., the Internet.

The computing system can include clients and servers. A client and server are generally remote from each other and typically interact through a communication network. The relationship of client and server arises by virtue of computer programs running on the respective computers and having a client-server relationship to each other. In some embodiments, a server transmits data, e.g., an HTML page, to a user device, e.g., for purposes of displaying data to and receiving user input from a user interacting with the device, which acts as a client. Data generated at the user device, e.g., a result of the user interaction, can be received at the server from the device.

While this specification contains many specific implementation details, these should not be construed as limitations on the scope of any invention or on the scope of what may be claimed, but rather as descriptions of features that may be specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially be claimed as such, one or more features from a claimed combination can in some cases be excised from the combination, and the claimed combination may be directed to a subcombination or variation of a subcombination.

Similarly, while operations are depicted in the drawings and recited in the claims in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system modules and components in the embodiments described above should not be understood as requiring such separation in all embodiments, and it should be understood that the described program components and systems can generally be integrated together in a single software product or packaged into multiple software products.

Particular embodiments of the subject matter have been described. Other embodiments are within the scope of the following claims. For example, the actions recited in the claims can be performed in a different order and still achieve desirable results. As one example, the processes depicted in the accompanying figures do not necessarily require the particular order shown, or sequential order, to achieve desirable results. In some cases, multitasking and parallel processing may be advantageous.

Further aspects of the invention are defined in the following clauses:
1. A computer-implemented method comprising:
   receiving data characterizing a molecule;
   processing a model input that comprises the data characterizing the molecule, using a retrosynthesis featurization machine learning model and in accordance with values of a set of retrosynthesis featurization machine learning model parameters, to generate a feature array comprising a plurality of features of a retrosynthesis tree for the molecule; and
   processing a model input that comprises the feature array comprising the plurality of features of the retrosynthesis tree for the molecule, using a synthesizability prediction machine learning model and in accordance with values of a set of synthesizability prediction machine learning model parameters, to generate a synthesizability score that characterizes a synthesis complexity of the molecule.
2. The method of clause 1, wherein the data characterizing the molecule comprises data defining a chemical structure of the molecule.
3. The method of clause 2, wherein the data defining the chemical structure of the molecule comprises a Simplified Molecular Input Line Entry System (SMILES) string.
4. The method of any one of clauses 1-3, wherein the retrosynthesis featurization machine learning model has been trained by operations comprising:
   obtaining a set of training examples, wherein each training example corresponds to a respective training molecule and comprises: (i) a training model input characterizing the training molecule, and (ii) a target feature array comprising a plurality of features of a retrosynthesis tree for the training molecule; and
   training the retrosynthesis featurization machine learning model on the set of training examples.
5. The method of clause 4, wherein a plurality of training examples in the set of training examples have been generated by performing operations comprising:
   obtaining a collection of baseline retrosynthesis trees;
   generating a plurality of new retrosynthesis trees by programmatically assembling baseline retrosynthesis trees from the collection of baseline retrosynthesis trees;
   generating a respective training example for each new retrosynthesis tree, comprising, for each new retrosynthesis tree:
      generating a training model input for the training example based on a training molecule represented by a root node of the retrosynthesis tree; and
      generating a target feature array for the training example by processing the new retrosynthesis tree to determine the plurality of features of the retrosynthesis tree for the training molecule.
6. The method of clause 5, wherein obtaining the collection of baseline retrosynthesis trees comprises:
   identifying the collection of baseline retrosynthesis trees by scraping a corpus of data using natural language processing techniques.
7. The method of any one of clauses 5-6, wherein programmatically assembling baseline retrosynthesis trees from the collection of baseline retrosynthesis trees comprises, for each new retrosynthesis tree:
   searching a space of possible combinations of baseline retrosynthesis trees having leaf nodes that each represent a molecule included in a predefined library of precursor molecules to identify the new retrosynthesis tree, wherein the search is performed to optimize an objective function that measures a penalty associated with the new retrosynthesis tree.
8. The method of clause 7, wherein searching the space of possible combinations of baseline retrosynthesis trees further comprises:
   identifying a first baseline retrosynthesis tree comprising a root node and one or more leaf nodes;
   identifying a plurality of second baseline retrosynthesis trees having root nodes that each represent a same molecule as a corresponding leaf node of the first baseline retrosynthesis tree;
   determining, for each of the plurality of second baseline retrosynthesis tree, a penalty that would result from attaching the second baseline retrosynthesis tree to the first baseline retrosynthesis tree at the corresponding leaf node of the first baseline retrosynthesis tree; and
   selecting a second baseline retrosynthesis tree for attachment to the first baseline retrosynthesis tree at each of the corresponding leaf nodes based on the penalties.
9. The method of clause 8, further comprising using dynamic programming to iteratively identify the first baseline retrosynthesis tree and the plurality of second baseline retrosynthesis trees for each of a set of root nodes and corresponding leaf nodes of the new retrosynthesis tree.
10. The method of any one of clauses 7-9, wherein the penalty characterizes one or more of: a depth of the new retrosynthesis tree, a measure of ease of procurement of the molecules represented by the nodes in the new retrosynthesis tree, or a yield of the reactions in a synthesis pathway represented by the new retrosynthesis tree.
11. The method of clause 4, wherein for a plurality of training examples in the set of training examples, the target feature array of the training example is generated by operations comprising:
   processing data characterizing the training molecule using a retrosynthesis planner to generate a predicted retrosynthesis tree for the training molecule; and
   processing the predicted retrosynthesis tree for the training molecule to generate the target feature array of the training example.
12. The method of clause 11, wherein processing data characterizing the training molecule using a retrosynthesis planner to generate a predicted retrosynthesis tree for the training molecule comprises:
   processing the data characterizing the training molecule using the retrosynthesis planner to generate a plurality of predicted retrosynthesis trees for the training molecule; and
   wherein processing the predicted retrosynthesis tree for the training molecule to generate the target feature array of the training example comprises:
      processing the plurality of predicted retrosynthesis trees to generate the target feature array of the training example.
13. The method of clause 12, wherein processing the plurality of predicted retrosynthesis trees to generate the target feature array of the training example comprises:
   processing each of the plurality of predicted retrosynthesis trees to generate a corresponding feature array; and
   aggregating the feature arrays for the plurality of predicted retrosynthesis trees to generate the target feature array.
14. The method of clause 12, wherein processing the plurality of predicted retrosynthesis trees to generate the target feature array of the training example comprises:
   selecting a set of at least one but fewer than all of the plurality of predicted retrosynthesis trees; and
   generating the target feature array based on only the selected predicted retrosynthesis trees.
15. The method of clause 14, wherein selecting the set of at least one but fewer than all of the plurality of predicted retrosynthesis trees comprises:
   ranking the plurality of retrosynthesis trees based on one or more criteria to select the set; or
   clustering the plurality of retrosynthesis trees to select the set based at least on a measure of diversity of synthesis.
16. The method of any one of clauses 11-15, wherein the retrosynthesis planner is implemented as a machine learning model having a set of machine learning model parameters that are trained by a machine learning training technique.
17. The method of clause any one of clauses 4-16, wherein training the retrosynthesis featurization machine learning model on the set of training examples comprises, for each training example:
   training the retrosynthesis featurization machine learning model to reduce a discrepancy between: (i) the target feature array of the training example, and (ii) a predicted feature array generated by processing the training model input of the training example using the retrosynthesis featurization machine learning model.
18. The method of any one of clauses 1-17, wherein the retrosynthesis featurization machine learning model comprises a neural network.
19. The method of any one of clauses 1-17, wherein the retrosynthesis featurization machine learning model comprises a graph neural network.
20. The method of clause 19, wherein processing the model input that comprises the data characterizing the molecule, using the retrosynthesis featurization machine learning model and in accordance with values of the set of retrosynthesis featurization machine learning model parameters, to generate the feature array comprising the plurality of features of the retrosynthesis tree for the molecule comprises:
   generating data defining a graph representing the molecule, wherein the graph comprises a set of nodes and a set of edges, wherein each edge in the set of edges connects a respective pair of nodes from the set of nodes, wherein each node in the set of nodes is associated with a respective set of node features;
   processing, for each node in the graph, the set of node features of the node using an encoder block of the graph neural network to generate an embedding of the node;
   processing the embeddings of the nodes in the graph by a plurality of message passing neural network layers of the graph neural network to generate a respective final embedding for each node in the graph; and
   processing the final embeddings of the nodes in the graph using a decoder block of the graph neural network to generate the feature array.
21. The method of any one of clauses 1-20, wherein the synthesizability prediction machine learning model has been trained by operations comprising:
   obtaining a set of training examples, wherein each training example corresponds to a respective training molecule and comprises: (i) a training model input comprising a feature array including a plurality of features of a retrosynthesis tree for the training molecule, and (ii) a target synthesizability score for the training molecule; and
   training the retrosynthesis featurization machine learning model on the set of training examples.
22. The method of clause 21, wherein training the synthesizability prediction machine learning model on the set of training examples comprises, for each training example:
   training the synthesizability prediction machine learning model to reduce a discrepancy between: (i) the target synthesizability score of the training example, and (ii) a predicted synthesizability score generated by processing the training model input of the training example using the synthesizability prediction machine learning model.
23. The method of any one of clauses 1-22, wherein the synthesizability prediction machine learning model comprises a support vector machine.
24. The method of any one of clauses 1-22, wherein the synthesizability prediction machine learning model comprises a differentiable machine learning model.
25. The method of clause 24, wherein the retrosynthesis featurization machine learning model and the synthesizability prediction machine learning model have been jointly trained by operations comprising:
   obtaining a set of training examples, wherein each training example corresponds to a respective training molecule and comprises: (i) a training model input comprising data characterizing the training molecule, and (ii) a target synthesizability score; and
   training the retrosynthesis featurization machine learning model and the synthesizability prediction machine learning model to reduce a discrepancy between: (i) the target synthesizability score of the training example, and (ii) a predicted synthesizability score.
26. The method of clause 25, wherein training the retrosynthesis featurization machine learning model and the synthesizability prediction machine learning model comprises:
   processing each of the training model inputs using the retrosynthesis featurization machine learning model to generate respective predicted feature arrays;
   processing each of the respective predicted feature arrays using the synthesizability prediction machine learning model to generate respective predicted synthesizability scores; and
   updating values of a set of retrosynthesis featurization machine learning model parameters and values of a set of synthesizability prediction machine learning model parameters in accordance with reducing the discrepancy between: (i) the respective target synthesizability score, and (ii) the respective predicted synthesizability scores.
27. The method of any one of clauses 1-26, further comprising using the synthesizability score to perform classifier guidance for a generative model that is configured to generate output molecules.
28. The method of clause 27, wherein the generative model is configured to generate a ligand molecule for binding to a target protein molecule.
29. The method of any one of clauses 27-28, wherein the generative model is implemented as a generative diffusion model.
30. The method of any one of clauses 27-29, wherein using the synthesizability score to perform classifier guidance for the generative model encourages the generative model to generate output molecules that have at least a threshold level of synthesizability.
31. The method of any one of clauses 27-30, wherein using the synthesizability score to perform classifier guidance for the generative model comprises modifying an intermediate layer representation of the generative model.
32. The method of any preceding clause, wherein the feature array comprises:
   a set of one or more features characterizing the retrosynthesis tree; and
   a set of one or more biophysical and biochemical properties of the molecule.
33. The method of any preceding clause, wherein the set of one or more features characterizing the retrosynthesis tree comprises one or more of: a depth indicative of a longest path from a root node of the retrosynthesis tree to any leaf node of the retrosynthesis tree, a number of nodes of the retrosynthesis tree, a probability of a reaction represented by the retrosynthesis tree, a reactivity indicator with reference to the retrosynthesis tree, a stability indicator with reference to the retrosynthesis tree, and a completeness of the retrosynthesis tree indicative of each molecule represented by a leaf node of the retrosynthesis tree being included in a predefined library of precursor molecules.
34. The method of any one of clauses 32-33, wherein the set of one or more biophysical and biochemical properties comprises one or more of: molecular weight, topological polar surface area, formal charge, number of hydron acceptors and/or donors, number of rotatable bonds, number of aromatic and aliphatic rings, number of carbon-carbon single bonds, number of heavy atoms, or number of heteroatoms.
35. A computer-implemented method comprising:
   receiving a collection of candidate molecules;
   generating a respective synthesizability score for each of the candidate molecules using the method of any one of clauses 1-34; and
   selecting one or more of the candidate molecules for physical synthesis based at least in part on the respective synthesizability scores.
36. The method of clause 35, further comprising physically synthesizing the one or more candidate molecules selected for synthesis.
37. The method of any one of clauses 35-36, further comprising performing physical experiments to determine one or more properties of the physically synthesized one or more candidate molecules.
38. A system comprising one or more computers and one or more storage devices storing instructions that are operable, when executed by the one or more computers, to cause the one or more computers to perform the method of any one of clauses 1-35.
39. One or more non-transitory computer storage media encoded with a computer program, the program comprising instructions that are operable, when executed by one or more computers, to cause the one or more computers to perform the method of any one of clauses 1-35.

## Claims

1. A computer-implemented method comprising:
receiving data characterizing a molecule;
processing a model input that comprises the data characterizing the molecule, using a retrosynthesis featurization machine learning model and in accordance with values of a set of retrosynthesis featurization machine learning model parameters, to generate a feature array comprising a plurality of features of a retrosynthesis tree for the molecule; and
processing a model input that comprises the feature array comprising the plurality of features of the retrosynthesis tree for the molecule, using a synthesizability prediction machine learning model and in accordance with values of a set of synthesizability prediction machine learning model parameters, to generate a synthesizability score that characterizes a synthesis complexity of the molecule.

2. The method of claim 1, wherein the data characterizing the molecule comprises data defining a chemical structure of the molecule, in particular wherein the data defining the chemical structure of the molecule comprises a Simplified Molecular Input Line Entry System (SMILES) string; and/or
wherein the set of one or more features characterizing the retrosynthesis tree comprises one or more of: a depth indicative of a longest path from a root node of the retrosynthesis tree to any leaf node of the retrosynthesis tree, a number of nodes of the retrosynthesis tree, a probability of a reaction represented by the retrosynthesis tree, a reactivity indicator with reference to the retrosynthesis tree, a stability indicator with reference to the retrosynthesis tree, and a completeness of the retrosynthesis tree indicative of each molecule represented by a leaf node of the retrosynthesis tree being included in a predefined library of precursor molecules.

3. The method of any one of claims 1-2, wherein the retrosynthesis featurization machine learning model has been trained by operations comprising:
obtaining a set of training examples, wherein each training example corresponds to a respective training molecule and comprises: (i) a training model input characterizing the training molecule, and (ii) a target feature array comprising a plurality of features of a retrosynthesis tree for the training molecule; and
training the retrosynthesis featurization machine learning model on the set of training examples.

4. The method of claim 3, wherein a plurality of training examples in the set of training examples have been generated by performing operations comprising:
obtaining a collection of baseline retrosynthesis trees;
generating a plurality of new retrosynthesis trees by programmatically assembling baseline retrosynthesis trees from the collection of baseline retrosynthesis trees;
generating a respective training example for each new retrosynthesis tree, comprising, for each new retrosynthesis tree:
generating a training model input for the training example based on a training molecule represented by a root node of the retrosynthesis tree; and
generating a target feature array for the training example by processing the new retrosynthesis tree to determine the plurality of features of the retrosynthesis tree for the training molecule.

5. The method of claim 4, wherein programmatically assembling baseline retrosynthesis trees from the collection of baseline retrosynthesis trees comprises, for each new retrosynthesis tree:
searching a space of possible combinations of baseline retrosynthesis trees having leaf nodes that each represent a molecule included in a predefined library of precursor molecules to identify the new retrosynthesis tree, wherein the search is performed to optimize an objective function that measures a penalty associated with the new retrosynthesis tree; in particular wherein the penalty characterizes one or more of: a depth of the new retrosynthesis tree, a measure of ease of procurement of the molecules represented by the nodes in the new retrosynthesis tree, or a yield of the reactions in a synthesis pathway represented by the new retrosynthesis tree

6. The method of claim 5, wherein searching the space of possible combinations of baseline retrosynthesis trees further comprises:
identifying a first baseline retrosynthesis tree comprising a root node and one or more leaf nodes;
identifying a plurality of second baseline retrosynthesis trees having root nodes that each represent a same molecule as a corresponding leaf node of the first baseline retrosynthesis tree;
determining, for each of the plurality of second baseline retrosynthesis tree, a penalty that would result from attaching the second baseline retrosynthesis tree to the first baseline retrosynthesis tree at the corresponding leaf node of the first baseline retrosynthesis tree; and
selecting a second baseline retrosynthesis tree for attachment to the first baseline retrosynthesis tree at each of the corresponding leaf nodes based on the penalties.

7. The method of claim 3, wherein for a plurality of training examples in the set of training examples, the target feature array of the training example is generated by operations comprising:
processing data characterizing the training molecule using a retrosynthesis planner to generate a predicted retrosynthesis tree for the training molecule; and
processing the predicted retrosynthesis tree for the training molecule to generate the target feature array of the training example; in particular
wherein processing data characterizing the training molecule using a retrosynthesis planner to generate a predicted retrosynthesis tree for the training molecule comprises:
processing the data characterizing the training molecule using the retrosynthesis planner to generate a plurality of predicted retrosynthesis trees for the training molecule; and
wherein processing the predicted retrosynthesis tree for the training molecule to generate the target feature array of the training example comprises:
processing the plurality of predicted retrosynthesis trees to generate the target feature array of the training example.

8. The method of claim 7, wherein processing the plurality of predicted retrosynthesis trees to generate the target feature array of the training example comprises:
processing each of the plurality of predicted retrosynthesis trees to generate a corresponding feature array; and
aggregating the feature arrays for the plurality of predicted retrosynthesis trees to generate the target feature array; or
wherein processing the plurality of predicted retrosynthesis trees to generate the target feature array of the training example comprises:
selecting a set of at least one but fewer than all of the plurality of predicted retrosynthesis trees; and
generating the target feature array based on only the selected predicted retrosynthesis trees.

9. The method of any one of claims 3-8, wherein training the retrosynthesis featurization machine learning model on the set of training examples comprises, for each training example:
training the retrosynthesis featurization machine learning model to reduce a discrepancy between: (i) the target feature array of the training example, and (ii) a predicted feature array generated by processing the training model input of the training example using the retrosynthesis featurization machine learning model.

10. The method of any one of claims 1-9, wherein the synthesizability prediction machine learning model has been trained by operations comprising:
obtaining a set of training examples, wherein each training example corresponds to a respective training molecule and comprises: (i) a training model input comprising a feature array including a plurality of features of a retrosynthesis tree for the training molecule, and (ii) a target synthesizability score for the training molecule; and
training the retrosynthesis featurization machine learning model on the set of training examples; in particular wherein training the synthesizability prediction machine learning model on the set of training examples comprises, for each training example:
training the synthesizability prediction machine learning model to reduce a discrepancy between: (i) the target synthesizability score of the training example, and (ii) a predicted synthesizability score generated by processing the training model input of the training example using the synthesizability prediction machine learning model.

11. The method of any one of claims 1-10, wherein the synthesizability prediction machine learning model comprises a differentiable machine learning model, and wherein the retrosynthesis featurization machine learning model and the synthesizability prediction machine learning model have been jointly trained by operations comprising:
obtaining a set of training examples, wherein each training example corresponds to a respective training molecule and comprises: (i) a training model input comprising data characterizing the training molecule, and (ii) a target synthesizability score; and
training the retrosynthesis featurization machine learning model and the synthesizability prediction machine learning model to reduce a discrepancy between: (i) the target synthesizability score of the training example, and (ii) a predicted synthesizability score; in particular
wherein training the retrosynthesis featurization machine learning model and the synthesizability prediction machine learning model comprises:
processing each of the training model inputs using the retrosynthesis featurization machine learning model to generate respective predicted feature arrays;
processing each of the respective predicted feature arrays using the synthesizability prediction machine learning model to generate respective predicted synthesizability scores; and
updating values of a set of retrosynthesis featurization machine learning model parameters and values of a set of synthesizability prediction machine learning model parameters in accordance with reducing the discrepancy between: (i) the respective target synthesizability score, and (ii) the respective predicted synthesizability scores.

12. The method of any one of claims 1-11, further comprising using the synthesizability score to perform classifier guidance for a generative model that is configured to generate output molecules, wherein the generative model is configured to generate a ligand molecule for binding to a target protein molecule, and wherein using the synthesizability score to perform classifier guidance for the generative model encourages the generative model to generate output molecules that have at least a threshold level of synthesizability.

13. A computer-implemented method comprising:
receiving a collection of candidate molecules;
generating a respective synthesizability score for each of the candidate molecules using the method of any one of claims 1-12; and
selecting one or more of the candidate molecules for physical synthesis based at least in part on the respective synthesizability scores.

14. A system comprising one or more computers and one or more storage devices storing instructions that are operable, when executed by the one or more computers, to cause the one or more computers to perform the method of any one of claims 1-13.

15. One or more non-transitory computer storage media encoded with a computer program, the program comprising instructions that are operable, when executed by one or more computers, to cause the one or more computers to perform the method of any one of claims 1-13.
